# EUROPEAN PATENT SPECIFICATION

(11) **EP 4 321 116 B1**
(45) Date of publication and mention of the grant of the patent: **14.01.2026**
(21) Application number: 23191165.2
(22) Date of filing: 11.08.2023
(51) Int. Cl.: A61B 17/34, A61M 1/04, A61M 25/06

(54) **PERCUTANEOUS ACCESS PATHWAY SYSTEM**
PERKUTANES ZUGANGSWEGSYSTEM
SYSTÈME DE VOIE D'ACCÈS PERCUTANÉ

(30) Priority: 11.08.2022 US 202263397006 P
(43) Date of publication of application: 14.02.2024
(73) Proprietor: Critical Innovations, LLC, Lawndale, CA 90260 (US)
(72) Inventor: DONALDSON, Ross I., California, 90260 (US); BUCHANAN, Oliver, California, 90260 (US); HAMDAN, Muhammed, California, 90260 (US); YESAYAN, Zhirair, California, 90260 (US); FISHER, Timothy, California, 90260 (US)
(74) Representative: HGF

(56) References cited:
- RU-C1- 2 082 445
- RU-U1- 193 743
- US-A- 5 226 426
- US-A1- 2013 310 750
- US-A1- 2017 182 229
- US-A1- 2020 022 688
- US-A1- 2021 106 344

## Description

### RELATED APPLICATION

The present application claims the benefit of U.S. Provisional Application No. 63/397,006 filed August 11, 2022.

### COMMONLY OWNED APPLICATIONS

This invention was made with government support under contract W81XWH-20-C-0096, "Donaldson Decompression Needle for automated diagnosis and treatment of tension pneumothorax," awarded by U.S. Army Medical Research Acquisition Activity (USAMRAA). The United States government has certain rights in the invention.

### TECHNICAL FIELD

The present disclosure relates generally to the field of medical devices, and more particularly, to devices and methods for forming and/or maintaining a percutaneous access pathway in a patient's body.

### BACKGROUND

A wide variety of diagnostic and therapeutic procedures involve the creation of a percutaneous access pathway into the body of a patient (e.g., a body cavity). General objectives of access systems are to maximize the chances of entry into the appropriate anatomical region while minimizing chances of injury during the establishment of such access.

The literature discloses various additional known methods and devices for forming and/or maintaining percutaneous access pathways. Known in the art are "Veress" or "Veress-type" needles (i.e., Veres), which have an outer needle having a sharp distal end and an inner probe with a blunt distal end that extends through the outer needle. **In** Veress needles, the inner probe is biased to force the blunt distal end of the probe beyond the sharp distal end of the outer needle. However, when the blunt distal end of the probe encounters dense material (e.g., abdominal wall), the probe is forced backwards and the sharp end of the needle is presented so that it can puncture the dense material. Once penetrating the denser material (e.g., abdominal wall) the biasing force again returns the blunt distal end of the probe beyond the sharp distal end of the outer needle. This mechanism can alert the user that they are in the appropriate anatomic space (e.g., through visual, tactile, and/or auditory means) and assist with prevention of injury to internal organs (e.g., intestines).

Veress needles are used for many procedures in veterinary and human medicine. For example, the art describes many uses to establish and/or maintain a percutaneous access pathway into the peritoneal cavity. This includes establishment of an access pathway to provide insufflation (e.g., of carbon dioxide and/or another gas) prior to laparoscopic surgery. For example, U.S. Pat. No. 5,421,821 to Janicki et al describes a Veress needle system and method for insufflating the abdominal cavity prior to laparoscopic surgery. Other example uses in conjunction with abdominal laparoscopic procedures include U.S. Pat. Nos. 5,376,082; 5,207,647; 5,514,111; 5,827,221; and 6,221,048 to Phelps; 5,660,883 to Scarfone, et al; and U.S. Pat. No. 5,098,388 to Kulkashi et al.

U.S. Pat. No. 5,300,046 to Scarfone, et al; 5,997,486 to Burek, et al; and, U.S. Pat. No. 5,725,506 to Freeman, et al describe similar Veress devices and methods to access the thoracic cavity (e.g., for thoracentesis). U.S. Pat. No. 5,334,159 to Turkel also describes a Veress thoracentesis needle having a direct passageway through the needle for withdrawing fluid into the pleural space. Additionally, Turkel describes subsequent modifications in U.S. Pat. No. 5,685,852, which is a similar Veress device modified for use as an epidural needle.

There are also several variations on Veress needles in the art. These include an improved hyper-sensitive detection mechanism for sensing when the blunt portion of the Veress is in contact with inner tissue (e.g., U.S. Pat. No. 6,447,483 to Steube, et al). Additionally, many commercially available Veress-type needles have one or more automatic check valves. These include the "Toramatic" devices (e.g., 1-55, 1-80, 1-VER) from Bioservice S.p.A. and Medax Medical Devices, "Safe-T-Centesis^{®} Catheter Drainage System" from CareFusion, and the "Pleura-safe^{®} Safety Thoracentesis Needle" from Allomed Medtech GmbH.

However, as aforementioned, there has been known and long-standing problems with Veress needles and their related technologies, which have unfortunate rates of "plunging" that can result in iatrogenic injury to underlying structures (i.e., entry-related injury) and/or lead to misplacement of the needle tip into an undesired location. Despite their long and documented history since the 1930s, existing Veress needle assemblies of the art have not overcome this important problem.

Previous to our work, such plunging was primarily attributed to user error in not stopping needle penetration immediately upon device indicator alert (i.e., failing to stop immediately upon change of indicator color or audible "click" of the needle). However, during the development of a device that automates the stopping process and thus removes this type of user error, it was discovered that at least a portion of plunging incidents occur not because of this user error, but instead due to the inherent physical characteristics of existing Veress technology. As demonstrated by our experiments, a previously unappreciated factor in Veress plunging is the locking (i.e., jamming) of the device mechanism when significant lateral pressure is applied to the needle. **In** practice, such lateral forces can be caused by a non-perpendicular approach to skin puncture, application of rotational force during insertion (e.g., twisting of the wrist), lateral tissue tension, and other related causes. This results in the probe becoming stuck while retracted within the needle. It is thus unable to correctly redeploy back to its starting position once through the denser material tissue (e.g., abdominal wall). This then causes a failure of the device to notify the user that the needle tip is in the position of interest once reached (e.g., peritoneal cavity), which may cause the user to incorrectly continue to advance the needle tip into underlying structures (i.e., "plunging").

Our investigations have revealed that Veress needles currently known to the art lock (i.e., jam) when significant lateral pressure is applied to the needle due to the inner probe becoming stuck or otherwise reducing its mobility when retracted within the needle. This may cause a delay or complete failure to notify the user that the relevant tissue has been pierced. This occurs when the needle is bent from forces applied laterally to the line of needle of entry, making the probe within it less mobile. For exiting Veress needles, not all securely hold the needle into the housing base, which exacerbates this problem. However, even for those that securely attach the needle to the housing, if the needle bends a sufficient amount, it can prevent easy probe movement in current technology.

As such, the percutaneous access devices and methods of the art have not before provided for an improved Veress-type device, especially one that prevents or reduces the chances of (e.g., increases the lateral force required to cause) device jamming when lateral force is applied to the needle. This leaves an important need for a device and method to do so.

Additionally, the literature discloses various known methods and devices for diagnosing tension pneumothorax in conjunction with the establishment of a percutaneous access pathway. It has been reported that providers may hear a short "whish" of air out of a needle when placed into the pleural cavity of a patient with tension pneumothorax. However, this is very subjective and difficult to hear environments with background noise (e.g., standard background noise, sirens, gunfire, hospital alarms) and their ear not immediately next to the needle hub. Additionally, it has previously been noted that some check valves used with a decompression needle can create an audible whistle when air rushes through (North American Rescue Simplified Pneumothorax Emergency Air Release Device (SPEAR) product information, https://www.narescue.com/spear-simplified-pneumothorax-emergency-air-release.html, Accessed 7/29/2022). However, no previous research has demonstrated that such a check valve reliably produces an auditory alarm (e.g., whistle), which reliably occurs when there is tension pneumothorax (i.e., true positive) and does not occur in common situations during which tension is not present (i.e., false positive), such as needle insertion, needle manipulation, patient movement, and/or cardiopulmonary resuscitation.

Document US 5 226 426 A describes a penetrating instrument including a tubular needle having a distal end with a sharp tip, a safety probe movable within the needle between an extended position protecting the needle tip and a retracted position exposing the needle tip and biased toward the extended position, and a portal sleeve surrounding the needle to establish communication with an anatomical cavity with penetration of tissue surrounding the cavity by the needle.

Document US 2013/310750 A1 describes a medical device including a portion similar to a Veress needle, useful for relief of tension pneumothorax.

Document US 2017/182229 A1 describes a method and device for simultaneously or near-simultaneously diagnosing and treating tension pneumothorax and/or hemothorax.

Document RU 2 082 445 C1 describes diagnostic puncture and micro-drainage of the pleural cavity performed with a non-rigid tube with several side holes at the inner end and a check valve at the outer end, made in the form of a body with a tense elastic membrane, partially fixed at one of its ends.

Document RU 193 743 U1 describes a device for surgical assistance with pneumothorax in the field and includes a bandage with a through central hole, the upper part of which is made with an airtight material, and the lower part having an adhesive layer.

Document US 2020/022688 A1 describes systems, methods and compositions relating to delivering synthetic polymer formulations to the body.

Document US 2021/106344 A1 describes an improved method and device for forming and/or maintaining a percutaneous access pathway,

### SUMMARY

The invention is defined by the independent claim. Further embodiments are recited in the dependent claims.

The present disclosure overcomes and substantially alleviates the deficiencies in the prior art by providing improved devices and methods for forming and/or maintaining a percutaneous access pathway. Under various embodiments, the initial percutaneous access pathway is formed via different methods and devices, which include the aforementioned techniques noted as background of the present disclosure. The provided assembly substantially reduces the possibility of iatrogenic injury while accessing and/or re-accessing a body space.

**In** many embodiments, the improved Veress needle is composed of a longitudinally extending hollow needle which is sharp at its distal end to allow for puncturing external tissue (e.g., abdominal wall) and extending into a cavity (e.g., abdominal cavity), a longitudinally extending hollow probe which extends through the hollow needle and has a blunt distal end with one or more openings, a spring (or other resilient biasing means) which is coupled to the probe and which resiliently biases the probe forward with respect to the hollow needle such that the blunt distal end of the probe extends past the sharp distal end of the needle, and a generally hollow housing which is fixed to the needle and which houses the resilient biasing means and permits relative movement of the probe relative to the housing.

Under many embodiments, the percutaneous access pathway is an improved Veress needle that is resistant to jamming (i.e., locking) of the device mechanism from lateral force applied to the needle. Under many embodiments, the Veress needle securely holds the needle in its housing base. Under various embodiments, this is achieved by ensuring there can be minimal movement between the needle and the housing body through: having a longer area of "capture" between the needle within the housing body (e.g., ≥1 cm; ≥1.5 cm; ≥2 cm; ≥2.5 cm); by injection overmolding of the needle base into the housing body and/or a component that securely attaches to the housing body; and/or by the addition of added securing features (e.g., screws) to connect to the housing body the needle and/or other features connected directly to the needle.

Under many embodiments, the percutaneous access pathway is an improved Veress needle resistant to jamming from lateral needle force that is achieved via a reduction in the flexibility of the needle body (i.e., reduced bend from a given lateral force). Under various embodiments, this reduction in needle flexibility is achieved by increasing the wall thickness of the needle, addition of a stabilizer that is in contact or near contact with the needle and provides added rigidity to the needle, using a material with reduced flexibility properties, and/or use of a non-circular shape that reduces needle flexibility in at least one direction.

Under many embodiments, the percutaneous access pathway is an improved Veress needle resistant to jamming from lateral needle force that is achieved via increased flexibility of the probe (i.e., increased bend from a given lateral force). Under various embodiments, this increase in probe flexibility is achieved by decreasing the wall thickness of the probe, using a material with increased flexibility properties, using a smaller external diameter of the probe in relation to the internal diameter of the needle to leave an increased gap between the two structures, and/or use of a non-circular shape that increases needle flexibility in at least one direction.

Under additional embodiments, the percutaneous access pathway is an improved Veress needle resistant to jamming from lateral needle force that is achieved via a change in the relationship between the needle and the probe (e.g., making the probe more flexible than the needle). Under various embodiments, this change in the relationship between the needle and the probe is achieved by a needle with a thicker wall diameter than the probe, by a needle consisting of a material that is less flexible than the probe material, a needle with a larger internal diameter in comparison to the external diameter of the probe (i.e., leaving a larger gap between the two), use of a lubricant or other friction-reducing agent between the inside of the needle and outside of the probe, and/or use of friction-reducing materials for the probe and/or needle.

Under many embodiments, the percutaneous access pathway has a means for automatically halting the forward movement of the device upon penetration into a body cavity (e.g., peritoneal cavity). Under many embodiments, the external movement of the probe in relation to the needle tip causes movement of the probe within the housing such that an external stabilizer that is flush with the patients skin can move in relation to the rest of the device only when the probe tip is in the proximal position in relation to the needle tip (e.g., while penetrating the abdominal wall), but will not move when the probe is in the extended distal position (e.g., once reaching the peritoneal cavity). Under many embodiments, this mechanism automatically prevents the user from penetrating the device too far into the body cavity (i.e., "plunging"). This assists the user in halting forward movement of the needle upon penetration into a body cavity (e.g., peritoneal cavity).

Under many embodiments, the percutaneous access pathway has a means to reset the external stabilizer (e.g., for use when reusing the device on another patient site, for use when initial deployment was unsuccessful in reaching the appropriate anatomic location, The resetting of the stabilizer allows it to return to a more distal position, to its near-original position, and/or to its original position. For example, during use the stabilizer is pushed proximally towards the user by the patient's body and resetting it returns it to a more distal position. If removed from the patient, resetting the device allows the stabilizer to return to a more distal position such that it again provides functionality upon reinsertion (e.g., can stop distal movement of the device once locked). In some examples not forming part of the claimed invention, this resetting occurs automatically once force is removed from the distal end of the stabilizer. In some embodiments, this resetting mechanism occurs via placing the removed device tip back onto the patient, at which time force on the distal probe tip combined with a stabilizer resilient biasing mechanism (e.g., a needle spring) causes the stabilizer to reset. In accordance with the claimed invention, this resetting mechanism occurs via the user interacting with a manual resetting mechanism (e.g., push button, twist button, and/or other user interface) that manually resets the stabilizer. In at least one embodiment, the manual resetting mechanism causes the lock to disengage with the stabilizer such that, biased by a stabilizer resilient biasing mechanism, it is then caused to reset.

In many embodiments, the access pathway device additionally includes a component for reversibly or irreversibly connecting to one or more attachment devices. In many of these embodiments, such a connecting component is a luer lock, while in others it is one of the many other medical connectors well known in the art (e.g., Y, barbed, stepped, bayonet, cross, compression, elbow, flare, funnel, giant-bore, large-bore, ISO 80369-3 ENFit^{®}, ISO 80369-6 NRFit^{™}, non-threaded, panel mount, pipe thread, quick disconnect, shielded, suction, T). In many of these embodiments, the device initially has a cap or other sealing mechanism on the connecting component (three-way stopcock), which is removed during use to allow connection to an attachment device. Such removal allows entrance to a pathway within the access device that ends within a body cavity when in use, which the cap or other sealing mechanism can reversibly block, thus preventing air and/or infection from entering the body cavity when in its closed position. In many of these embodiments, this cap or other sealing mechanism is airtight and in many it is non-pierceable. In many of these embodiments, the cap or other sealing mechanism prevents easy access to an internally sterile space within the access pathway device while in place. In many of these embodiments, tubing and/or a machine may be connected to the access pathway to administer and/or remove gases and/or liquids (e.g., peritoneal insufflation; medication administration; suction).

By way of exemplification, in some embodiments, the percutaneous access pathway is utilized to access the peritoneal cavity. Under some of these embodiments, the percutaneous access pathway consists of an improved Veress needle (e.g., resistant to jamming from lateral needle force); a means for automatically halting the forward movement of the device upon penetration into a body cavity (e.g., peritoneal cavity); and a component (e.g., luer lock) for reversibly or irreversibly connecting to one or more attachment devices. In many of these embodiments, the device is provided sterile within a barrier packaging. In many of these embodiments, tubing, syringe, and/or a machine may be connected to the access pathway to administer and/or remove gases and/or liquids (e.g., peritoneal insufflation; medication administration; ascitic fluid removal; peritoneal dialysis).

By way of exemplification, in some embodiments, the device is utilized to treat and/or diagnose tension pneumothorax. Under some of these embodiments, the percutaneous access pathway consists of a Veress needle mechanism; a means for automatically halting the forward movement of the device upon penetration into a body cavity (e.g., pleural cavity); and a check valve mechanism. Under many of these embodiments, the Veress needle mechanism is improved to be resistant to jamming from lateral needle force. Under many of these embodiments, the check valve mechanism is removable (e.g., in conjunction with a component [e.g., luer lock] for reversibly connecting to one or more attachment), non-removable, and/or integrated into the body of the device. **In** many of these embodiments, the device is provided sterile within barrier packaging (e.g., hard case with a tamper-evident seal).

Under some embodiments, the means for automatically halting the forward movement of the device upon penetration into a body cavity allow the device to be utilized in a wider range of anatomic locations. For example, current needles for the treatment of tension pneumothorax are supposed to be inserted only in very specific locations (i.e., single points of entry) due to concern of injuring underlying organs if the needle is placed too deep (i.e., plunges). Example locations widely acknowledged in the prior art are the second intercostal space at the midclavicular line and/or the fourth or fifth intercostal space at the anterior axillary or axillary line. Under some embodiments, the percutaneous access pathway is a needle device to treat and/or diagnose tension pneumothorax that can be inserted over a wider chest area (in comparison to prior art), over the majority of the chest (e.g., anterior and/or posterior chest), within a set area drawn over the anterior chest (e.g., a "box" drawn from the mid-clavicular line to the axillary line medial-laterally, from the second intercostal space to the inferior sternum superior-inferiorly), and/or bilaterally. Under some embodiments, there is a method of using a needle device to treat and/or diagnose tension pneumothorax as aforementioned, in contrast with current clinical practice and existing art.

Under some embodiments, the device is formed such that it provides an auditory alarm that serves to diagnose tension pneumothorax (i.e., of increased pressure within the pleural cavity). Under some embodiments, the auditory alarm (e.g., whistle) is provided by the opening of a check valve (e.g., duckbill valve). Under some embodiments, the auditory alarm (e.g., whistle) occurs when there is tension pneumothorax (i.e., true positive). Under some of these embodiments, the pressure required to trigger the auditory alarm is >0.1 PSI, >0.11 PSI, >0.14 PSI, >0.15 PSI, >0.2 PSI, and/or >0.27 PSI. It is reminded that 1 PSI corresponds to 6894.76 Pa.

Under some embodiments, the auditory alarm (e.g., whistle) does not occur in common situations during which tension is not present (i.e., false positive), such as needle insertion, needle manipulation, patient movement, and/or cardiopulmonary resuscitation.

Under some of these embodiments, an auditory alarm produces a true negative diagnosis of tension pneumothorax at reliable rates (i.e., lack device whistle in the absence of tension pneumothorax). Under some of these embodiments, an auditory alarm produces a true positive diagnosis of tension pneumothorax at reliable rates (i.e., positive device whistle in the presence of tension pneumothorax). Under some of these embodiments, at a 1-meter distance from the sound source there is a detectable distinguishable acoustic indication sound level between 1000 Hz to 3500 Hz. Under some of these embodiments, at a 1-meter distance from the sound source there is a detectable distinguishable acoustic indication sound level between 1000 Hz to 3500 Hz of greater than 20 Hz at 6dB delta above baseline. Under some of these embodiments, at a 1-meter distance from the sound source there is a detectable distinguishable acoustic indication at and/or around 1610 Hz greater than the following decibels above baseline (i.e., delta): 0, 5, 10, 15, 20, and/or 25. Under some of these embodiments, at a 1-meter distance from the sound source there is a detectable distinguishable acoustic indication at and/or around 3216 Hz greater than the following decibels above baseline (i.e., delta): 0, 5, 10, 15, 20, 25, 30, and/or 35. Under some of these embodiments, at a 1-meter distance from the sound source there is a detectable distinguishable acoustic indication at and/or around 8046 Hz greater than the following decibels above baseline (i.e., delta): 0, 5, 10, 15, 20, 25, 30, 35 and/or 39.

Under some embodiments, the device is formed such that the automatic check valve does not need to be removed during device insertion, to ensure that it functionally prevents air ingress throughout device placement. This includes if the device was inadvertently used on a healthy lung (i.e., one without tension pneumothorax present at time of utilization). This is in contrast to some prior art (e.g., SPEAR needle) that has a check valve attached to the device, but that fails to prevent air ingress into the body during a step when the needle is removed from the catheter and before replacement of the check valve on the catheter itself. This can result in air ingress during this period when the check valve not in place.

**In** many embodiments, the device anchors, stabilizes, and/or secures the percutaneous access pathway to the body (e.g., via securing the access pathway port to the skin and/or deeper structures). Examples include through sutures, staples, glue, gum, hydrogel, sticky-pad, and/or tape, tension from an expanded catheter within the body wall, adhesive that holds the catheter, port, and/or a larger stabilization pad onto the skin, and/or, expansion of one or more balloon(s) within the body cavity, within the percutaneous access pathway, and/or externally. In various embodiments, the access pathway device is anchored to make the percutaneous access pathway perpendicular to the skin, at a non-perpendicular angle, and/or adjustable so as to allow movement to a desired angle. In some embodiments, this securing means is placed over the device in a subsequent step to reduce chances of dislodgement (e.g., during transport or other patient movement). This securing means can interact with the needle body, the device body, and/or the stabilizer as well as the patient (e.g., skin) to hold the two together.

Under some embodiments, the needle is a stiff metallic tube with a sharp cutting and/or grinding edge that is not readily deformable. Examples of cutting and/or grinding edge embodiments include beveled tips (with one, two, three, or more cutting angles), angled tips, tips with abrasive grit, tips with spaced teeth, and tips related to known art for cutting surfaces. However, under some embodiments, the needle is a generally hollow tube (e.g., blunt). Under some embodiments, the probe is blunt, sharp, generally hollow, and/or generally solid. Under some embodiments, there are various numbers of holes in the probe. Under some embodiments, there are no holes in the probe. Under some embodiments, there is a removable or non-removable stylet that is located within the probe.

In some embodiments, the access pathway device is covered partially or fully with additional material(s) that can provide additional benefits when in contact with the body tissue. Examples include means to increase and/or decrease the cross-sectional area, to reduce friction and/or the chances of tissue being pinched, to decrease the chances of infection (e.g., antimicrobial properties), to prevent clotting, and/or to have drug-releasing properties (e.g., analgesic or other anti-pain medications).

In some embodiments, there is an additional catheter outside the needle of the access pathway device, which in some embodiments is removable (e.g., leaving the catheter within the patient). In some embodiments, this is in a manner similar to those disclosed in U.S. Pat. No. 7,229,433 to Mullen. Under various embodiments, the catheter is similar to a standard catheter, Penrose drain, pigtail catheter, chest tube, tracheostomy tube, endotracheal tube, venous catheter, arterial catheter, thoracentesis tube, paracentesis tube, abscess drainage, other medical tube, and/or other catheter for placement into a body cavity.

In some embodiments, the percutaneous access pathway is used for insufflation, thoracentesis, thoracostomy, paracentesis, arthrocentesis, tracheostomy, laparoscopy, laparotomy, lumbar puncture, cricothyroidotomy, abscess drainage and/or empyema drainage, central venous catheter, peripheral venous catheter, arterial catheter placement, ventriculostomy, and/or any medical procedure draining air and/or fluid and/or placing a catheter into a body cavity, hollow organ, vessel, and/or potential space.

In some embodiments, one device fits all patients. In others, part or all of the device is differently sized for different subgroups so that the appropriately sized device can be chosen for different subgroups based on, for example, weight, age, gender, length, pre-determined size categories (e.g., Broselow scale), and/or other indicators. Under various embodiments, differently sized components come together in a kit, with means for determining proper.

In some embodiments, the device includes a means for protecting the user from a needle stick injury when removing the device from the patient. Under this embodiment, such means can be arranged from any one of the many self-blunting needle mechanisms for intravenous catheters or phlebotomy needles that are well known in the art. Under some embodiments, this means is achieved by locking of the probe in its distally extended "blunt" configuration.

Under different embodiments, different portions of the device are disposable and/or non-disposable. In some embodiments, the device is inexpensively manufactured with all of the device designed to be disposed of after a single use. Parts may be made of metal or plastic or other suitable material. Under many embodiments, the device is compatible and/or safe for use in an x-ray, CT, and/or MRI environment. Under various embodiments, different parts are composed of a radio-opaque material and/or contain radio-opaque markers.

In many embodiments, the device is an improvement on prior art. For example, in some embodiments, the device includes a pierceable or non-predicable port, is caused to spin by use of a drill, connects with a device for delivering polymer, it is used for the treatment and/or diagnosis of tension pneumothorax, and/or is used in conjunction with one or more springs and/or resiliently biasing mechanisms so as to provide a hyper-sensitive detection mechanism to detect the lung (e.g., as in U.S. Pat. No. 6,447,483 to Steube, et al).

There have been illustrated and described herein methods and devices for forming and/or maintaining a percutaneous access pathway. While particular embodiments of the disclosure have been described, it is not intended that the disclosure be limited thereto, as it is intended that the disclosure be as broad in scope as the art will allow and that the specification be read likewise.

From the foregoing, it can be seen that the present disclosure provides an effective means for forming and/or maintaining a percutaneous access pathway within animals, especially humans. In various embodiments, the device is used to form and/or maintain a percutaneous access pathway into different body cavities. These include pathways into the abdomen, as well as the chest (e.g., pleural cavity, heart), retroperitoneal, cranium, trachea, abscess, artery, bladder, bone, collection of fluid (e.g., empyema, ascites, pleural, other effusion), organ, skull, trachea, vein, vessel, and/or, other body cavity. Although the example of the abdomen (e.g., peritoneal cavity) or chest (e.g., pleural cavity) has at times been used to illustrate the disclosure, it should not limit its scope as it could also similarly be used to access other body cavities and/or spaces. The device can also similarly be used with any other surgical procedure where an auto-stopping needle would be of benefit.

Moreover, it should also be apparent that the device can be made in varying lengths, sizes and capacities, and the precise composition of the device may be varied appropriately to treat adults, children, and infants. While the device has been described with a certain degree of particularity, it is manifest that many changes may be made in the details of construction and the arrangement of components without departing from the scope of this disclosure. It is understood that the disclosure is not limited to the embodiments set forth herein for purposes of exemplification and that elements of certain embodiments can be combined with elements of other embodiments. Additional objects, advantages, and novel features of the disclosure will be set forth in the description which follows, and will become apparent to those skilled in the art upon examination of the following detailed description and figures. It should be understood that not all of the features described need be incorporated into a given system or method.

### BRIEF DESCRIPTION OF THE DRAWINGS

FIG. 1 is an isometric view of the access pathway in accordance with an embodiment of the disclosure, as assembled prior to use.
FIG. 2 is a side view of the access pathway in accordance with an embodiment of the disclosure, as assembled prior to use.
FIG. 3 is a cross-sectional view of the access pathway in accordance with an embodiment of the disclosure, shown as assembled prior to insertion into a body.
FIG. 4 is a cross-sectional view of the access pathway of Figure 4, shown upon penetration of body tissue (e.g., abdominal wall).
FIG. 5 is a cross-sectional view of the access pathway of Figure 4, shown upon entrance into a body cavity (e.g., peritoneal cavity).
FIG. 6 is a side view of the access pathway in accordance with an embodiment of the disclosure including a check valve, as assembled prior to use.
FIG. 7 is a graph of example acoustic characteristics of an auditory alarm for the diagnosis of tension pneumothorax, when used on a live animal.

### DETAILED DESCRIPTION OF THE INVENTION

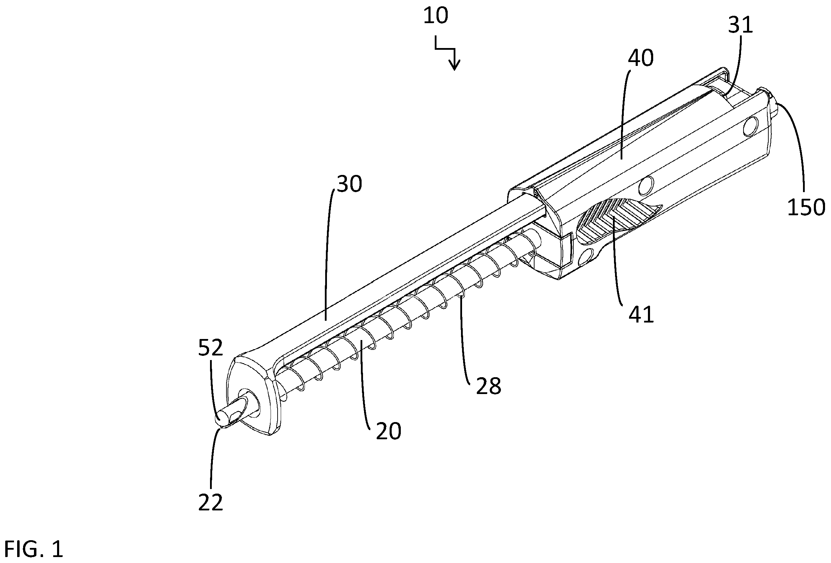

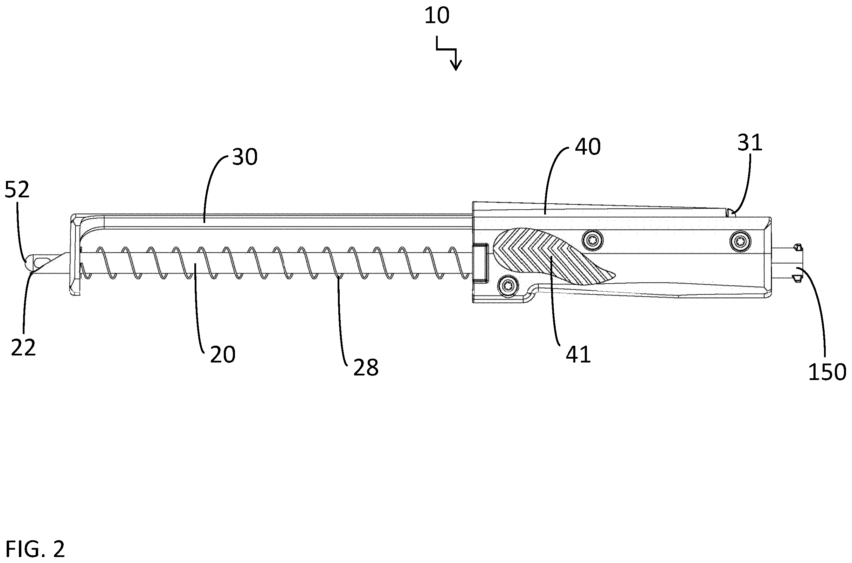

Referring to the drawings, Figures **1** and **2** illustrate one embodiment of part of the present disclosure. For ease of reference, distal shall refer to the end of the device farthest away from the user, while proximal shall refer to the end of the device closest to the user. Under this embodiment, stabilizer **30** initially extends distally from housing **40** along the tract of needle **20,** to provide a mechanism for automatically halting the forward movement of needle tip **22** and probe tip **52** upon penetration into a body cavity (e.g., peritoneal cavity). Also seen is connector **150,** which in this embodiment is a luer lock connector that can connect to standard syringe fittings, ports, 3-way stopcock, insufflation tubing, and/or drainage tubing. Also seen are finger grips **41,** for improved grip, and stabilizer phalange **31,** which prevents stabilizer **30** from falling out of the device once assembled, due to an interaction with housing **40.** Finally, needle spring **28** surrounds needle **20** and to apply force on stabilizer **30** when compressed.

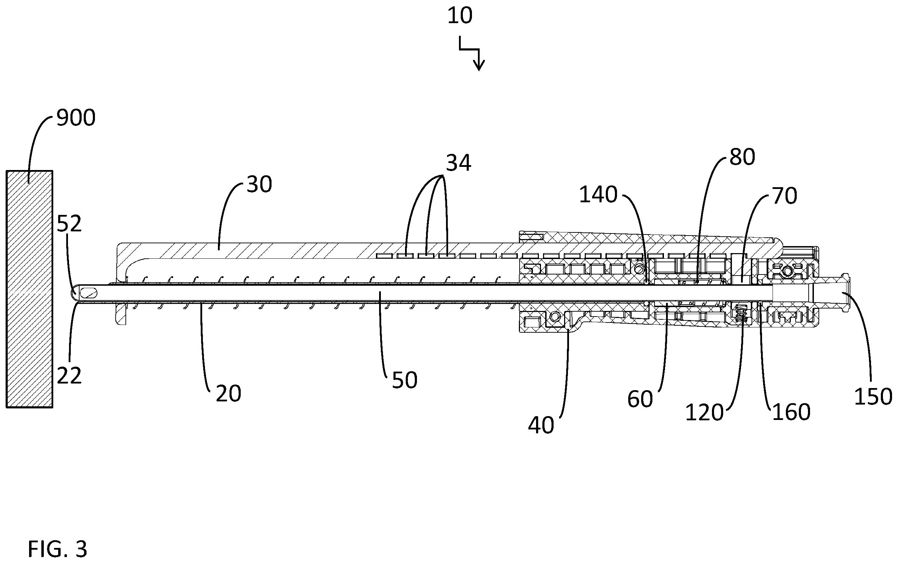

Moving now to FIG. **3****,** an embodiment of part of the present disclosure is shown in a cross-sectional view, before insertion into the example of a human body. Under this embodiment, stabilizer **30** initially extends distally from housing **40** along the tract of needle **20.** Probe **50** is connected to key **60,** such that they move together as one unit and are able to move proximally and distally within housing **40.** Axial spring **80** biases probe **50** and key **60** to their distal position, with probe tip **52** of probe **50** protruding out distally past needle tip **22** of needle **20.** Lock **70** is capable of perpendicular movement, to engage or disengage with grooves **34** on stabilizer **30.** In this initial position, as there is no force on probe tip **52** of probe **50,** key **60** (biased by axial spring **80)** is in its distal position. As key **60** is not engaged with lock **70,** locking spring **120** keeps lock **70** engaged with grooves **34** on stabilizer **30.** This in turn inhibits movement of stabilizer **30** in relation to housing **40.**

Additionally shown are O-rings **140** and **160** that, together with the other related components, ensure an airtight space between the distal end of needle **20,** through probe **50,** to connector **150.** This allows administration of fluid or gas through the device into the body (e.g., insufflation, medication administration) and/or allows for the exit of gas and/or fluid. Specifically, the inclusion of O-ring **140** in this embodiment, ensures that there is no leak between probe **50** and needle **20,** such that the contagious space distally includes needle **20** and not just the probe **50,** which is advantageous in many scenarios.

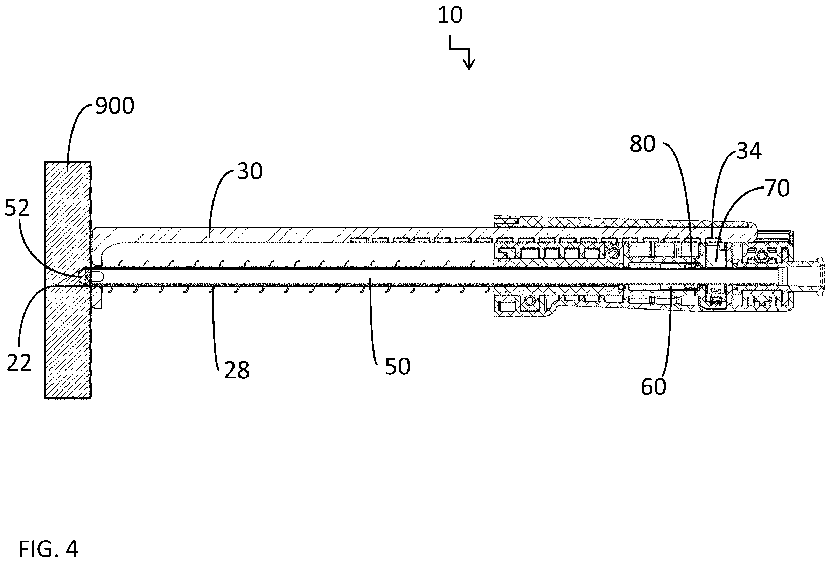

FIG **4** shows an embodiment of the access pathway device upon contact with body wall **900.** When a user pushes the device onto body wall **900** the countervailing force overcomes axial spring **80** and pushes probe tip **52** (partially hidden in this image inside body wall **900)** proximally in reference to the rest of the device, which further exposes needle tip **22** (also partially hidden in this image inside body wall **900).** This also causes probe **50** and key **60** to move proximally in reference to housing **40,** which in turn causes key **60** to engage with lock **70** so that lock **70** becomes no longer in contact with grooves **34.** Thus, when the device encounters resistance from body wall **900,** although stabilizer **30** is flush with skin (as assured by needle spring **28),** it is mobile so that the device can move distally into the body cavity.

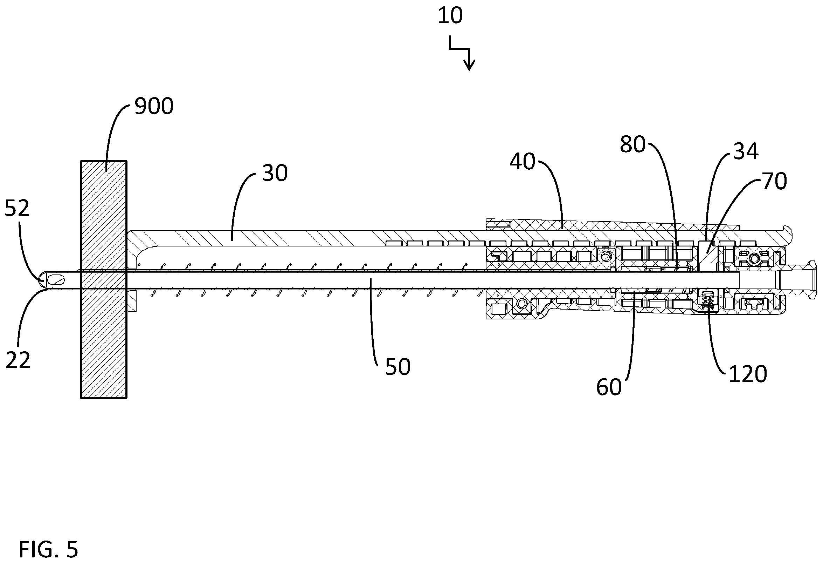

FIG **5** shows an embodiment of the access pathway after passing through body wall **900** and into a body cavity. Once probe tip **52** has passed through body wall **900** and has reached a body cavity, axial spring **80** is free to move key **60** and thus probe **50** distally in reference to housing **40.** This disengages key **60** from lock **70,** which (pushed by lock locking spring **120)** then reengages with grooves **34** to inhibit the movement of stabilizer **30** in relation to housing **40.** This, in turn, prevents needle tip **22** from moving further into the cavity, thus minimizing the chances of injuring distal structures.

Embodiments disclosed herein can further provide for an improved Veress-type device that prevents or reduces the chances of (e.g., increases the lateral force required to cause) device jamming when lateral force is applied to the needle.

In embodiments, the device is resistant to jamming (i.e., locking) of the device mechanism from lateral force applied to the needle because the Veress needle securely holds the needle in its housing base. For example, this can be achieved by ensuring there is minimal movement between the needle and the housing body through, for example, one or more of having a longer area of "capture" between the needle within the housing body (e.g., ≥1 cm; ≥1.5 cm; ≥2 cm; ≥2.5 cm), injection overmolding of the needle base into the housing body, including a component that securely attaches to the housing body amd the addition of added securing features (e.g., screws) to connect to the housing body the needle and/or other features connected directly to the needle.

In embodiments, a device that is resistant to jamming from lateral needle force is achieved via a reduction in the flexibility of the needle body (i.e., reduced bend from a given lateral force). For example, this reduction in needle flexibility can be achieved by, for example, one ore more of increasing the wall thickness of the needle, addition of a stabilizer that is in contact or near contact with the needle and provides added rigidity to the needle, using a material with reduced flexibility properties, and use of a non-circular shape that reduces needle flexibility in at least one direction.

In embodiments, a device that is resistant to jamming from lateral needle force is achieved via increased flexibility of the probe (i.e., increased bend from a given lateral force). For example, this increase in probe flexibility can be achieved by, for example, one or more of decreasing the wall thickness of the probe, using a material with increased flexibility properties, using a smaller external diameter of the probe in relation to the internal diameter of the needle to leave an increased gap between the two structures, and use of a non-circular shape that increases needle flexibility in at least one direction.

In embodiments, a device that is resistant to jamming from lateral needle force can be achieved via a change in the relationship between the needle and the probe (e.g., making the probe more flexible than the needle). For example, this change in the relationship between the needle and the probe can be achieved, for example, by one or more of a needle with a thicker wall diameter than the probe, a needle consisting of a material that is less flexible than the probe material, a needle with a larger internal diameter in comparison to the external diameter of the probe (i.e., leaving a larger gap between the two), use of a lubricant or other friction-reducing agent between the inside of the needle and outside of the probe, and use of friction-reducing materials for the probe and/or needle.

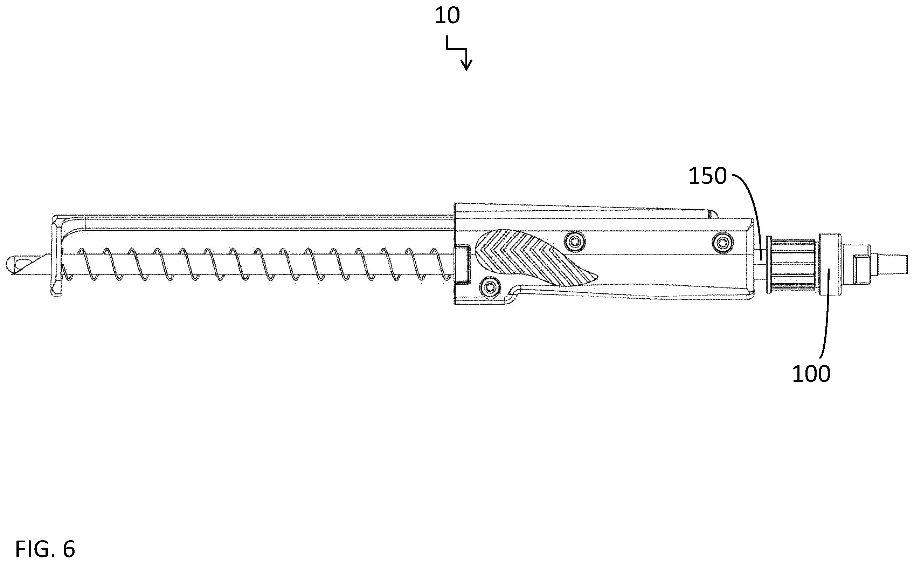

Figure 6 shows another embodiment in accordance with an embodiment of the disclosure, wherein the access pathway device includes check valve **100** as assembled prior to use. In some embodiments, this is a duck bill valve, while in others another automatic check valve is utilized, such as a flapper check valve, ball in socket check valve, umbrella check valve, v-tip needle check valve, and/or other automatic check valve. Additionally, in some embodiments more than one check valve is utilized. In some embodiments, check valve **100** is reversibly attached to connector **150,** while in others it is irreversibly attached. In some embodiments, check valve **100** provides an auditory alarm of tension pneumothorax based solely on air escaping the body of the patient through the check valve without any other component configured to cause audible noise indicative of such an alarm.

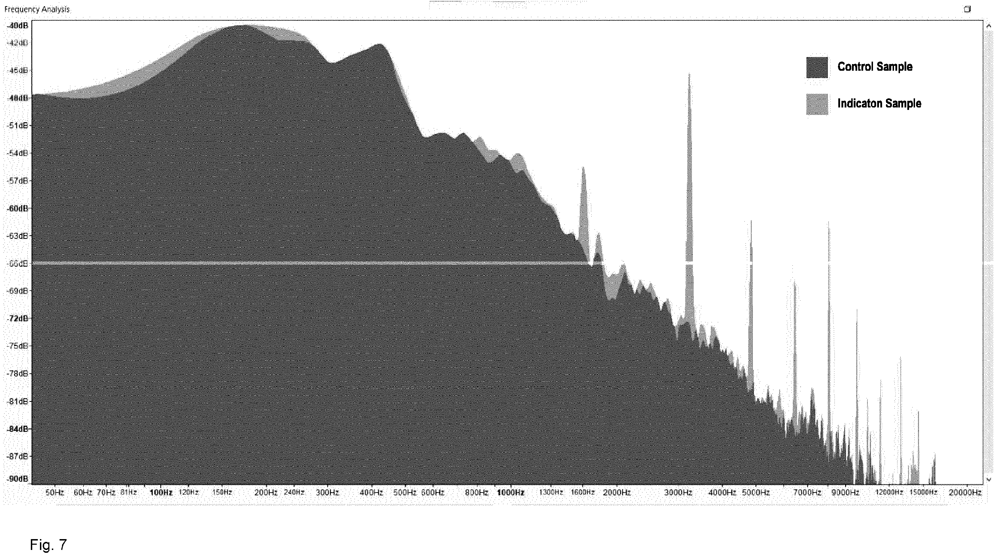

Figure 7 shows an example graph of the example acoustic characteristics of an auditory alarm for the diagnosis of tension pneumothorax, when used on a live animal, to particularly describe certain embodiments of the disclosure. These are intended for illustrative purposes only, since modifications and variations will be apparent to those skilled in the art and the auditory alarm is not present for all embodiments. The graph illustrates the results of a series of experiments to study the physical properties of an auditory alarm for tension pneumothorax. Devices with an auditory alarm were inserted into live animals, either with or without tension pneumothorax present (induced by inserting CO2 under pressure into the pleural space). Then, audio equipment was used to measure the diagnostic characteristics. There was no alarm issued in the absence of tension pneumothorax. In the presence of tension pneumothorax, the device consistently produced an auditory alarm, which is illustrated in this figure. There were noticeable increases in decibels in the device intervention group as compared to background noise at and/or around the following Hz levels: 1000, 1550, 1700, 1800-1900, 3200, and/or 4900.

In embodiments, a device for forming and/or maintaining an access pathway into a body of a patient can include a housing defining an open interior and having a proximal end and a distal end. The housing can include one or more openings. A generally hollow needle can extend distally from the distal end of the housing and have a cutting distal end. A probe can be slidably disposed within the generally hollow needle and have a blunt distal end and one or more openings adjacent to the distal end. The device can include means for biasing the distal end of the probe into a position distal to the distal cutting end of the hollow needle such that a force on the distal end of the probe can overcome the bias to move the probe proximally relative to the hollow needle. A mechanical lock can be configured to automatically halt distal movement of the hollow needle and probe in response to the distal end of the probe returning to the position distal to the distal cutting end of the hollow needle upon the hollow needle entering a body cavity of a patient. The needle can be sufficiently less flexible than the probe to inhibit bending of the needle relative to the probe when lateral forces are applied to the needle.

In some embodiments, the needle has a thicker wall diameter than a wall diameter of the probe.

In some embodiments, the needle comprises a material that is less flexible than a material comprising the probe.

In some embodiments, the device further includes a stabilizer configured to provide counterforce against device advancement.

In some embodiments, the device further includes a connecting component configured to connect to one or more attachment devices.

In some embodiments, the device is configured to allow for gas insulation through it into a body cavity.

In some embodiments, the device further includes a means to reset the stabilizer to a more distal position after initial use.

In embodiments, a device for forming and/or maintaining an access pathway into a body of a patient can include a housing defining an open interior and having a proximal end and a distal end. The housing can include one or more openings. A generally hollow needle can extend distally from the distal end of the housing and have a cutting distal end. A probe can be slidably disposed within the generally hollow needle and have a blunt distal end and one or more openings adjacent to the distal end. The device can include means for biasing the distal end of the probe into a position distal to the distal cutting end of the hollow needle such that a force on the distal end of the probe can overcome the bias to move the probe proximally relative to the hollow needle. A mechanical lock can be configured to automatically halt distal movement of the hollow needle and probe in response to the distal end of the probe returning to the position distal to the distal cutting end of the hollow needle upon the hollow needle entering a body cavity of a patient. A stabilizer can be configured to provide counterforce against device advancement and a resetting mechanism can be configured to reset the stabilizer to a more distal position relative to the distal end of the probe after initial use.

In some embodiments, the resetting mechanism causes the mechanical lock to disengage with the stabilizer such that, biased by a stabilizer resilient biasing mechanism, it is caused to reset when the distal end of the probe is placed onto the patient.

In some embodiments, the resetting mechanism includes a manually actuated input.

In some embodiments, the manually actuated input is a button.

In some embodiments, the stabilizer resilient biasing mechanism is a spring disposed around the needle that interfaces with the stabilizer.

In some embodiments, the resetting mechanism automatically resets the stabilizer once force is removed from a distal end of the stabilizer.

In some embodiments, the more distal position is an original position of the stabilizer relative to the distal position of the probe prior to use.

In embodiments, a device for forming and/or maintaining an access pathway into a body of a patient can include a housing defining an open interior and having a proximal end and a distal end. The housing can include one or more openings. A generally hollow needle can extend distally from the distal end of the housing and have a cutting distal end. A probe can be slidably disposed within the generally hollow needle and have a blunt distal end and one or more openings adjacent to the distal end. The device can include means for biasing the distal end of the probe into a position distal to the distal cutting end of the hollow needle such that a force on the distal end of the probe can overcome the bias to move the probe proximally relative to the hollow needle. A mechanical lock can be configured to automatically halt distal movement of the hollow needle and probe in response to the distal end of the probe returning to the position distal to the distal cutting end of the hollow needle upon the hollow needle entering a body cavity of a patient. A check valve can be disposed with a fluid flow path through the device and configured to provide an auditory alarm when air from within the body of the patient exits the check valve via the fluid flow path.

In some embodiments, the check valve is configured to provide the auditory alarm without the use of any other auditory component.

In some embodiments, the check valve is configured such that the auditory alarm reliably occurs when there is tension pneumothorax and does not occur in situations during which tension pneumothorax is not present.

In some embodiments, at a 1-meter distance from the sound source the auditory alarm has a detectable distinguishable acoustic indication sound level between 1000 Hz to 3500 Hz.

Various embodiments of systems, devices, and methods have been described herein. These embodiments are given only by way of example and are not intended to limit the scope of the disclosure. It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the disclosure.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

The invention is defined by the appended claims.

## Claims

1. A device for forming and/or maintaining an access pathway into a body of a patient, comprising:
a housing (40) defining an open interior and having a proximal end and a distal end, the housing including one or more openings;
a generally hollow needle (20) extending distally from the distal end of the housing, the generally hollow needle having a cutting distal end (22);
a probe (50) slidably disposed within the generally hollow needle, the probe having a blunt distal end (52) and one or more openings adjacent to the distal end;
means for biasing (80) the distal end of the probe into a position distal to the distal cutting end of the hollow needle such that a force on the distal end of the probe can overcome the bias to move the probe proximally relative to the hollow needle;
a mechanical lock (70) configured to automatically halting distal movement of the hollow needle and probe in response to the distal end of the probe returning to the position distal to the distal cutting end of the hollow needle upon the hollow needle entering a body cavity of a patient; and
a stabilizer (30) configured to provide counterforce against device advancement; and **characterised by**
a manual resetting mechanism configured to reset the stabilizer to a more distal position relative to the distal end of the probe after initial use, the manual resetting mechanism causing the stabilizer to be reset via a user interacting with a manually actuated input.

2. The device of claim 1, wherein the manual resetting mechanism causes the mechanical lock to disengage with the stabilizer such that, biased by a stabilizer resilient biasing mechanism, it is caused to reset when the distal end of the probe is placed onto the patient.

3. The device of claim 1, wherein the manually actuated input is a button.

4. The device of any preceding claim, further comprising a connecting component (150) configured to connect to one or more attachment devices.

5. The device of any preceding claim, wherein the device is configured to allow for gas insufflation through it into a body cavity.

## Patentansprüche

1. Vorrichtung zum Herstellen und/oder Aufrechterhalten eines Zugangswegs in einen Körper eines Patienten, umfassend:
ein Gehäuse (40), das einen offenen Innenraum definiert und ein proximales Ende und ein distales Ende aufweist, wobei das Gehäuse eine oder mehrere Öffnungen beinhaltet;
eine im Allgemeinen hohle Nadel (20), die sich distal von dem distalen Ende des Gehäuses erstreckt, wobei die im Allgemeinen hohle Nadel ein schneidendes distales Ende (22) aufweist;
eine Sonde (50), die verschiebbar innerhalb der im Allgemeinen hohlen Nadel angeordnet ist, wobei die Sonde ein stumpfes distales Ende (52) und eine oder mehrere Öffnungen benachbart zu dem distalen Ende aufweist;
Mittel zum Vorspannen (80) des distalen Endes der Sonde in eine Position distal zu dem distalen schneidenden Ende der hohlen Nadel, sodass eine Kraft an dem distalen Ende der Sonde die Vorspannung überwinden kann, um die Sonde proximal relativ zu der hohlen Nadel zu bewegen;
eine mechanische Verriegelung (70), die dazu konfiguriert ist, eine distale Bewegung der hohlen Nadel und der Sonde als Reaktion darauf automatisch anzuhalten, dass das distale Ende der Sonde in die Position distal zu dem distalen schneidenden Ende der Hohlnadel zurückkehrt, wenn die hohle Nadel in einen Körperhohlraum eines Patienten eintritt; und
einen Stabilisator (30), der dazu konfiguriert ist, eine Gegenkraft gegen eine Vorwärtsbewegung der Vorrichtung bereitzustellen; und gekennzeichnet durcheinen manuellen Rückstellmechanismus, der dazu konfiguriert ist, den Stabilisator nach dem ersten Gebrauch in eine distalere Position relativ zu dem distalen Ende der Sonde zurückzustellen, wobei der manuelle Rückstellmechanismus bewirkt, dass der Stabilisator durch einen Benutzer, der mit einer manuell betätigten Eingabe interagiert, zurückgestellt wird.

2. Vorrichtung nach Anspruch 1, wobei der manuelle Rückstellmechanismus bewirkt, dass die mechanische Verriegelung aus dem Stabilisator ausrückt, sodass sie, durch einen elastischen Vorspannmechanismus des Stabilisators vorgespannt, dazu veranlasst wird, sich zurückzusetzen, wenn das distale Ende der Sonde auf den Patienten platziert wird.

3. Vorrichtung nach Anspruch 1, wobei die manuell betätigte Eingabe eine Taste ist.

4. Vorrichtung nach einem vorhergehenden Anspruch, ferner umfassend eine Verbindungskomponente (150), die dazu konfiguriert ist, sich mit einer oder mehreren Anschlussvorrichtungen zu verbinden.

5. Vorrichtung nach einem vorhergehenden Anspruch, wobei die Vorrichtung dazu konfiguriert ist, eine Gasinsufflation durch sie hindurch in eine Körperhöhle zuzulassen.

## Revendications

1. Dispositif permettant de former et/ou maintenir une voie d'accès dans le corps d'un patient, comprenant :
un boîtier (40) définissant un intérieur ouvert et possédant une extrémité proximale et une extrémité distale, le boîtier comprenant une ou plusieurs ouvertures ;
une aiguille généralement creuse (20) s'étendant de manière distale depuis l'extrémité distale du boîtier, l'aiguille généralement creuse possédant une extrémité distale coupante (22) ;
une sonde (50) disposée de manière coulissante à l'intérieur de l'aiguille généralement creuse, la sonde possédant une extrémité distale émoussée (52) et une ou plusieurs ouvertures adjacentes à l'extrémité distale ;
un moyen pour solliciter (80) l'extrémité distale de la sonde dans une position distale par rapport à l'extrémité de coupe distale de l'aiguille creuse de sorte qu'une force sur l'extrémité distale de la sonde puisse surmonter la sollicitation pour déplacer la sonde de manière proximale par rapport à l'aiguille creuse ;
un verrou mécanique (70) conçu pour arrêter automatiquement le déplacement distal de l'aiguille creuse et de la sonde en réponse au retour de l'extrémité distale de la sonde dans la position distale par rapport à l'extrémité de coupe distale de l'aiguille creuse lorsque l'aiguille creuse pénètre dans une cavité corporelle d'un patient ; et
un stabilisateur (30) conçu pour fournir une contre-force contre l'avancée du dispositif ; et
**caractérisé par** un mécanisme de réinitialisation manuelle conçu pour réinitialiser le stabilisateur dans une position plus distale par rapport à l'extrémité distale de la sonde après une utilisation initiale, le mécanisme de réinitialisation manuelle provoquant la réinitialisation du stabilisateur par un utilisateur interagissant avec une entrée actionnée manuellement.

2. Dispositif selon la revendication 1, ledit mécanisme de réinitialisation manuelle amenant le verrou mécanique à se retirer du stabilisateur de sorte que, sollicité par un mécanisme de sollicitation élastique du stabilisateur, il soit amené à se réinitialiser lorsque l'extrémité distale de la sonde est placée sur le patient.

3. Dispositif selon la revendication 1, ladite entrée actionnée manuellement étant un bouton.

4. Dispositif selon l'une quelconque des revendications précédentes, comprenant en outre un composant de raccordement (150) conçu pour se raccorder à un ou plusieurs dispositifs de fixation.

5. Dispositif selon l'une quelconque des revendications précédentes, ledit dispositif étant conçu pour permettre une insufflation de gaz à travers celui-ci dans une cavité corporelle.
